# EUROPEAN PATENT APPLICATION

(11) **EP 4 710 853 A1**
(43) Date of publication of application: **18.03.2026**
(21) Application number: 24921084.0
(22) Date of filing: 04.02.2024
(51) Int. Cl.: A61B 5/11

(54) **SENSING APPARATUS FOR ACQUIRING MOTION SIGNAL OF TARGET OBJECT AND WEARABLE DEVICE**

(71) Applicant: Shenzhen Shokz Co., Ltd., Shenzhen, Guangdong 518108 (CN)
(72) Inventor: DENG, Wenjun, Shenzhen, Guangdong 518108 (CN); YUAN, Yongshuai, Shenzhen, Guangdong 518108 (CN)
(74) Representative: Wang, Bo
(86) International application number: PCT/CN2024/075852
(87) International publication number: WO 2025/161031

(57) **Abstract**

Provided are a sensing apparatus (100) for acquiring a motion signal of a target object, and a wearable device comprising the sensing apparatus (100). The sensing apparatus (100) comprises a first sensitive unit (120) arranged on a first side (110-a) of a flexible substrate (110) facing the target object. By means of a first conductive channel (111) arranged on the flexible substrate (110), an electrical signal generated by the first sensitive unit (120) is conducted to a target position (A), wherein the target position (A) is farther away from the target object than the first side (110-a), such that a connection position between the sensing apparatus (100) and an external circuit can be away from the first side (110-a), allowing the first side (110-a) of the sensing apparatus (100) facing the target object to remain flat, avoiding uneven deformation of the first sensitive unit (120) on the first side (110-a), and thus improving operational stability of the sensing apparatus (100). Moreover, the flatness of the first side (110-a) can also reduce foreign body sensation during the wearing of the sensing apparatus (100) and enhance the wearing comfort of the sensing apparatus (100).

## Description

### TECHNICAL FIELD

The present disclosure relates to the field of sensing technology, and in particular, to a sensor device and a wearable device for acquiring a motion signal of a target object.

### BACKGROUND

With the increasing popularity of wearable devices, flexible and stretchable wearable sensor devices suitable for integration into smart clothing (e.g., garments, pants, gloves, shoes, or the like) are being applied more widely. In specific scenarios, a connection between the sensor device and an external circuit needs to be considered. However, the connection may affect the reliability and wearing comfort of the sensor device.

Accordingly, there is a need to provide a sensor device and a wearable device for acquiring a motion signal of a target object, with high operational stability and improved wearing comfort.

### SUMMARY

One or more embodiments of the present disclosure provide a sensor device for acquiring a motion signal of a target object. The sensor device includes: a flexible substrate including a first side surface facing the target object; and a first sensing unit configured to detect the target object and generate an electric signal. The first sensing unit is disposed on the first side surface of the flexible substrate. The flexible substrate is provided with a first conductive channel. The first conductive channel is configured to transmit the electric signal generated by the first sensing unit to a target position, and the target position is farther away from the target object than the first side surface.

One or more embodiments of the present disclosure further provide a wearable device for acquiring a motion signal of a target object. The wearable device includes the sensor device as described above.

### BRIEF DESCRIPTION OF THE DRAWINGS

The present disclosure will be further described by way of exemplary embodiments, which will be described in detail through the drawings. The embodiments are not limiting. In the embodiments, identical reference numerals refer to identical structures, wherein:
FIG. 1 is a schematic diagram illustrating an exemplary structure of a sensor device according to some embodiments of the present disclosure;
FIG. 2 is a schematic diagram illustrating another exemplary structure of a sensor device according to some embodiments of the present disclosure;
FIG. 3 is a schematic diagram illustrating another exemplary structure of a sensor device according to some embodiments of the present disclosure;
FIG. 4 is a schematic diagram illustrating another exemplary structure of a sensor device according to some embodiments of the present disclosure;
FIG. 5 is a schematic diagram illustrating another exemplary structure of a sensor device according to some embodiments of the present disclosure;
FIG. 6 is a schematic diagram illustrating another exemplary structure of a sensor device according to some embodiments of the present disclosure; and
FIG. 7 is a schematic diagram illustrating another exemplary structure of a sensor device according to some embodiments of the present disclosure.

### DETAILED DESCRIPTION

To more clearly illustrate the technical solutions of the embodiments of the present disclosure, the accompanying drawings required for describing the embodiments will be briefly introduced below. Obviously, the accompanying drawings in the following description are merely some examples or embodiments of the present disclosure. Those of ordinary skill in the art may apply the present disclosure to other similar scenarios based on these accompanying drawings without creative efforts. It should be understood that these exemplary embodiments are provided only to enable relevant technical personnel to better understand and implement the present disclosure, and are not intended to limit the scope of the present disclosure in any way. Unless obviously obtained from the context or the context illustrates otherwise, the same numeral in the drawings refers to the same structure or operation.

As shown in the present disclosure and the claims, unless the context clearly indicates an exception, the terms "a", "an", "one", and/or "the" are not limited to the singular form and may also include the plural form. In general, the terms "comprise," "comprises," and/or "comprising," "include," "includes," and/or "including," merely prompt to include steps and elements that have been clearly identified, and these steps and elements do not constitute an exclusive listing. The methods or devices may also include other steps or elements. The term "based on" is "based at least in part on." The term "one embodiment" means "at least one embodiment"; the term "another embodiment" means "at least one other embodiment".

In the description of the present disclosure, it should be understood that the orientation or positional relationships indicated by terms such as "front", "rear", "ear hook", and "rear hook" are based on the orientation or positional relationships shown in the accompanying drawings. These terms are used only to facilitate the description of the present disclosure and simplify the description, and do not indicate or imply that the referred device or element must have a specific orientation or be constructed and operated in a specific orientation. Therefore, these terms should not be construed as limiting the present disclosure.

Furthermore, the terms "first" and "second" are used for descriptive purposes only, and should not be construed as indicating or implying relative importance or implicitly specifying the quantity of the indicated technical features. Thus, features defined by "first" and "second" may explicitly or implicitly include at least one such feature. In the description of the present disclosure, the meaning of "a plurality of" is at least two, e.g., two, three, etc., unless otherwise explicitly and specifically defined.

In the present disclosure, unless otherwise explicitly specified and defined, terms such as "install", "connected", "connect", and "fixed" should be understood broadly. For example, the connection may be a fixed connection, a detachable connection, or an integral connection. The connection may be a mechanical connection, an electrical connection, or a direct connection. The connection may also be an indirect connection through an intermediate medium, or an internal communication between two elements or an interaction relationship between two elements, unless otherwise explicitly defined. Those of ordinary skill in the art may understand the specific meanings of the above terms in the present disclosure based on specific situations.

Some embodiments of the present disclosure provide a sensor device. The sensor device includes a first sensing unit disposed on a first side surface of a flexible substrate facing a target object. By transmitting an electric signal generated by the first sensing unit to a target position through a first conductive channel provided on the flexible substrate, in which the target position is farther away from the target object than the first side surface, a connection position between the sensor device and an external circuit may be located away from the first side surface, so that the first side surface of the sensor device facing the target object may remain flat, a non-uniform deformation of the first sensing unit on the first side surface may be avoided, and operational stability of the sensor device may be improved. Meanwhile, keeping the first side surface flat can also reduce a foreign-body sensation when the sensor device is worn, and the wearing comfort of the sensor device can be improved.

Some embodiments of the present disclosure provide a wearable device for acquiring a motion signal of a target object. The wearable device includes the above-described sensor device. The wearable device may have relatively high operational stability and wearing comfort.

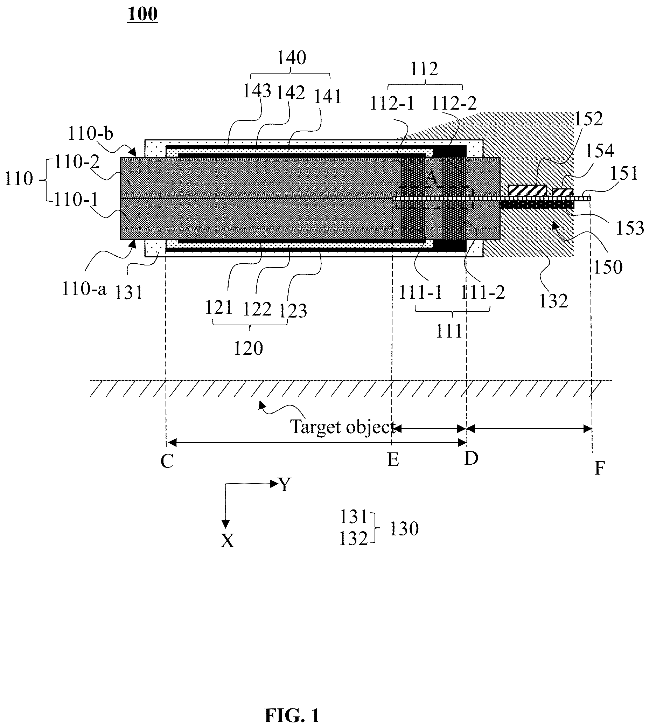

FIG. 1 is a schematic diagram illustrating an exemplary structure of a sensor device according to some embodiments of the present disclosure. As shown in FIG. 1, in some embodiments of the present disclosure, a sensor device 100 is provided. The sensor device 100 includes a flexible substrate 110 and a first sensing unit 120. The flexible substrate 110 serves as a mounting main body of the sensor device 100, and the first sensing unit 120 is mounted on the flexible substrate 110.

The first sensing unit 120 is configured to detect a target object and generate an electric signal. In some embodiments, the target object refers to an object from which the sensor device 100 acquires a motion signal. In some embodiments, the target object may include, but is not limited to, a human body, a portion of the human body, a movable object, etc. For example, the target object may be a finger of the human body, a palm of the human body, a motion-simulation mechanical arm, etc.

In some embodiments, the first sensing unit 120 may deform correspondingly in response to a motion of the target object, thereby generating a corresponding electric signal. By processing and analyzing the electric signal, motion information of the target object, such as a motion process or a motion posture, may be obtained. In some embodiments, the first sensing unit 120 may also directly read a related signal of the target object. Merely by way of example, the first sensing unit 120 may include a collection electrode. When the target object is the human body, the collection electrode may fit the skin of the human body, so that a electromyographic signal of the human body may be collected as an electric signal. When the target object is a mechanical structure, the collection electrode may be connected to a control circuit of the mechanical structure, so that a signal in the control circuit may be collected as the electric signal.

In some embodiments, the first sensing unit 120 may be disposed on a first side surface 110-a of the flexible substrate 110 facing the target object, so that a motion of the target object may be directly transmitted to the first sensing unit 120 to cause a deformation of the first sensing unit 120, such that the deformation of the first sensing unit 120 may better correspond to the motion of the target object, enabling the sensor device 100 to achieve a relatively high operational accuracy.

In some embodiments, the first sensing unit 120 may include, but is not limited to, a capacitive structure, a resistive structure, an inductive structure, etc. More descriptions regarding the above structures may be found in FIGs. 1-7 and related descriptions thereof.

In some embodiments, the flexible substrate 110 includes a second side surface 110-b opposite to the first side surface 110-a. The sensor device may further include a second sensing unit disposed on the second side surface 110-b (e.g., a second sensing unit 140 shown in FIG. 1). By providing the second sensing unit, the second sensing unit may also acquire information of the target object and generate an electric signal correspondingly. The electric signal generated by the second sensing unit may be combined with and compared against the electric signal generated by the first sensing unit, so that the measurement accuracy of the sensor device can be improved. For example, by performing differential processing on the electric signals generated by the first sensing unit and the second sensing unit, a noise signal generated due to wrinkling of the flexible substrate 110 can be eliminated. In some embodiments, a structure of the second sensing unit may be the same as or different from a structure of the first sensing unit. In some embodiments, to reduce a difference between the electric signal generated by the second sensing unit and the electric signal generated by the first sensing unit, so as to improve the operational stability and measurement accuracy of the sensor device, the structure of the second sensing unit may be the same as the structure of the first sensing unit. More descriptions regarding the second sensing unit may be found in FIGs. 1-6 and related descriptions thereof, and details are omitted herein.

The flexible substrate 110 may provide a mounting and fixing platform for other components (e.g., the first sensing unit 120) of the sensor device 100. In some embodiments, the flexible substrate 110 may have elasticity, so that the flexible substrate 110 may deform and bend correspondingly with a movement of the target object, thereby accurately reflecting the movement of the target object.

In some embodiments, a material of the flexible substrate 110 may include an insulating elastic material, such as Polydimethylsiloxane (PDMS), silica gel, or Thermoplastic polyurethanes (TPU). By adopting the insulating elastic material, the flexible substrate 110 may have both insulation and elasticity, so that an influence on the deformation of the first sensing unit 120 can be reduced, and an interference with the electric signal generated by the first sensing unit 120 can be avoided.

In some embodiments, the flexible substrate 110 is provided with a first conductive channel 111. One end of the first conductive channel 111 is electrically connected to the first sensing unit 120, and another end of the first conductive channel 111 extends to the target position (e.g., a region A shown in FIG. 1). The first conductive channel 111 is configured to transmit the electric signal generated by the first sensing unit 120 to the target position (as shown in FIG. 1).

Referring to FIG. 1, in some embodiments, a direction in which the sensor device 100 points toward the target object is set as an X-direction, and an extending direction of the flexible substrate 110 is set as a Y-direction. The first conductive channel 111 is disposed to extend along the X-direction.

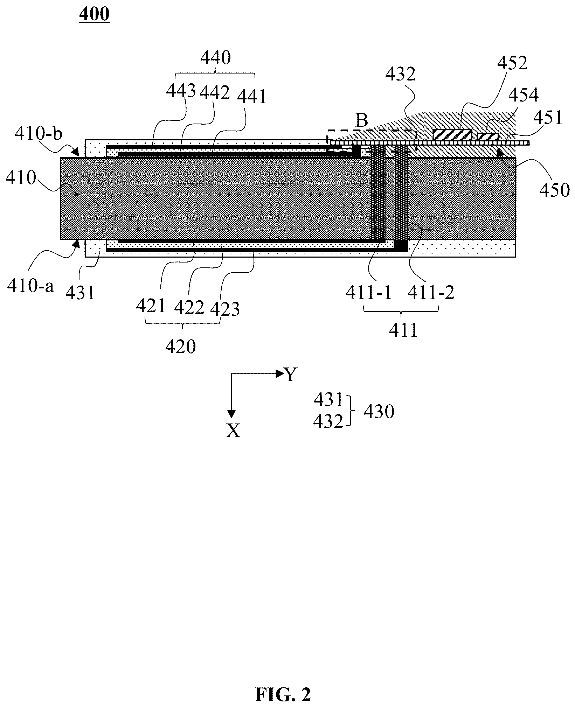

In some embodiments, the target position refers to a position configured for connecting another circuit (e.g., a processing circuit 152 shown in FIG. 1 or an external circuit), so that the electric signal generated by the first sensing unit 120 is transmitted to a corresponding circuit for processing or output. In some embodiments, the target position is farther away from the target object than the first side surface 110-a, i.e., a distance between the target position and the target object in the X-direction is greater than a distance between the first side surface 110-a and the target object in the X-direction, so that the first side surface 110-a can remain flat, a non-uniform deformation of the first sensing unit 120 can be avoided, and the operational stability of the sensor device 100 can be improved. Meanwhile, a foreign-body sensation when the sensor device 100 is worn can be reduced, and the wearing comfort of the sensor device 100 can be improved. For example, the target position may be located on the second side surface 110-b of the flexible substrate 110 away from the target object, as shown at a region B in FIG. 2, FIG. 4, and FIG. 6. As another example, the target position may be located between the first side surface 110-a and the second side surface 110-b, as shown at the region A in FIG. 1, FIG. 3, and FIG. 5.

In some embodiments, the flexible substrate 110 may be provided with a first hole portion (not shown in the figure), and the first hole portion may be filled with a flexible conductive material to form the first conductive channel 111, so that the first conductive channel 111 may deform to match a deformation of the flexible substrate 110 and avoid hindering a deformation of the first sensing unit 120. In some embodiments, the flexible substrate 110 may also be directly provided with a flexible conductor (e.g., a wire), and the flexible conductor directly forms a corresponding conductive channel.

In some embodiments, the flexible conductive material refers to a material having both conductivity and elasticity. In some embodiments, the flexible conductive material may include, but is not limited to, conductive silica gel, graphene, carbon nanotubes, metal nanowires, etc.

In some embodiments, the first hole portion may be processed and cut on the flexible substrate 110 by laser cutting or mechanical cutting.

If a size of the first hole portion is excessively large, an amount of the flexible conductive material may be relatively large, resulting in a relatively high cost. In addition, because the flexible conductive material may be different from the material of the flexible substrate 110, the first conductive channel 111 may interfere with the deformation of the flexible substrate 110 and the deformation of the first sensing unit 120. If the size of the first hole portion is excessively small, the processing difficulty may be relatively high, and the conduction performance of the first conductive channel 111 may be relatively poor, which may affect a transmission of the electric signal generated by the first sensing unit 120.

To save the cost, reduce an influence of the first conductive channel 111 on the deformation of the flexible substrate 110 and the deformation of the first sensing unit 120, and ensure the transmission performance of the electric signal of the first conductive channel 111, in some embodiments, a diameter of the first hole portion may be in a range of 0.7 mm to 1.3 mm. In some embodiments, to further save the material cost, the diameter of the first hole portion may be in a range of 0.8 mm to 1.2 mm. In some embodiments, to further reduce processing difficulty, the diameter of the first hole portion may be in a range of 0.9 mm to 1.1 mm. Merely by way of example, the diameter of the first hole portion is 1 mm.

In some embodiments, the sensor device 100 may further include an output circuit 150 disposed at the target position, and the output circuit 150 may receive the electric signal generated by the first sensing unit 120 and process or output the electric signal. Accordingly, the target position may be a position of the output circuit 150 in the X-direction or a position at which the sensing unit is connected to the output circuit.

In some embodiments, the first conductive channel 111 forms a first end portion (not shown in the figure) on the first side surface 110-a. The first end portion is electrically connected to an electrode of the first sensing unit 120. The first conductive channel 111 forms a second end portion (not shown in the figure) at the target position. The second end portion is electrically connected to the output circuit 150. The electric signal generated by the first sensing unit 120 is transmitted to the output circuit 150 through the first conductive channel 111, more descriptions regarding the transmission may be found in FIGs. 1-6 and related descriptions thereof.

In some embodiments, as the target position changes, the position of the output circuit 150 also changes accordingly. For example, when the target position is located at a middle portion between the first side surface 110-a and the second side surface 110-b of the flexible substrate 110 (i.e., the region A), the output circuit 150 is correspondingly disposed at a middle portion of the flexible substrate 110, as shown in FIG. 1, FIG. 3, and FIG. 5. As another example, when the target position is located at the second side surface 110-b of the flexible substrate 110 (i.e., the region B), the output circuit 150 is correspondingly disposed on the second side surface 110-b, as shown in FIG. 2, FIG. 4, and FIG. 6.

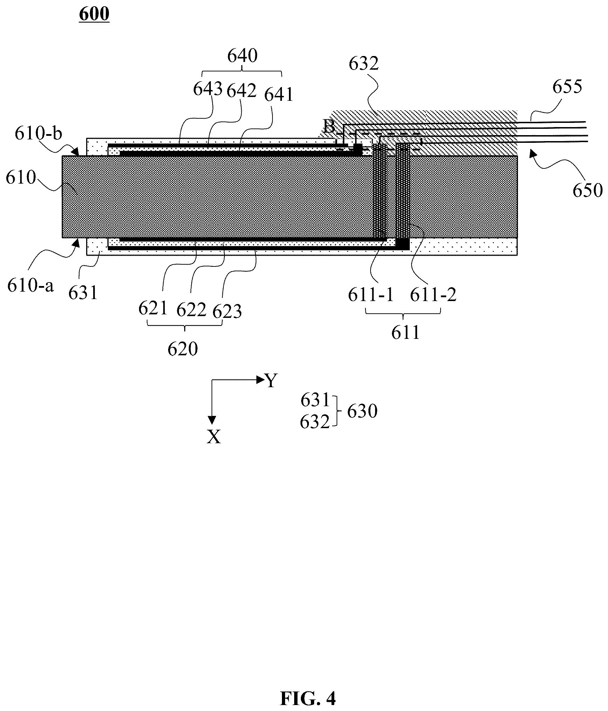

In some embodiments, the output circuit 150 may include a circuit board (e.g., a circuit board 151 shown in FIG. 1) or a wire (e.g., a wire 655 shown in FIG. 4). The circuit board is configured to receive the electric signal generated by the first sensing unit 120 and process or output the electric signal. By directly processing (e.g., filtering and denoising, combining with other electric signals, or performing time-domain or frequency-domain analysis to predict a variation trend) the electric signal generated by the first sensing unit 120, the sensor device 100 may operate without being connected to a processor, and the signal output by the sensor device 100 may directly represent the motion of the target object, or the sensor device 100 may output the electric signal after preliminary processing, so as to facilitate subsequent further processing. The wire may directly output the electric signal generated by the first sensing unit 120, and an arrangement position and a routing direction of the wire are flexible and variable, which can reduce an internal structural complexity of the sensor device 100 and simplify a difficulty of installation design.

In some embodiments, the circuit board is configured to connect to the external circuit (e.g., through a pad) to output the electric signal generated by the first sensing unit 120. In some embodiments, other components (e.g., the processing circuit 152) may also be provided on the circuit board to process the first sensing unit 120.

In some embodiments, when the output circuit 150 is disposed at the middle portion between the first side surface 110-a and the second side surface 110-b of the flexible substrate 110, the circuit board (e.g., the circuit board 151) extends into an interior of the flexible substrate 110 (as shown in FIG. 1). To avoid the circuit board from hindering the deformation of the flexible substrate 110 and the deformation of the first sensing unit 120, the circuit board may adopt a flexible printed circuit (FPC).

In other embodiments, when the output circuit 150 is disposed on the second side surface 110-b of the flexible substrate 110, the circuit board is disposed outside the flexible substrate 110 (e.g., a circuit board 451 shown in FIG. 2 is located outside a flexible substrate 410). In this way, the second sensing unit disposed on the second side surface does not need to be connected to the output circuit through a conductive channel formed on the flexible substrate 110, thereby avoiding forming excessive conductive channels on the flexible substrate 110, which can simplify the production process and improve the overall elasticity of the flexible substrate 110. In some embodiments, when the circuit board 151 of the output circuit 150 is located outside the flexible substrate 110, the circuit board may adopt a rigid printed circuit board (PCB) or an FPC.

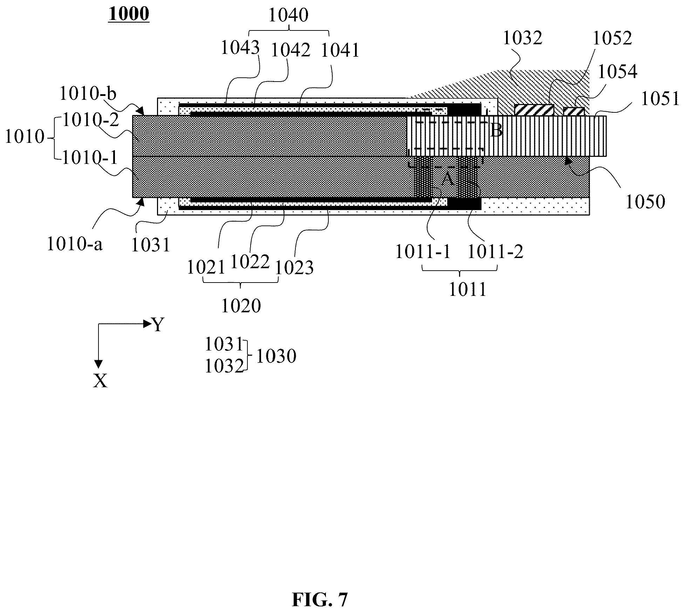

In still other embodiments, the output circuit may correspond to two different target positions simultaneously, and the first sensing unit and the second sensing unit may transmit their corresponding electric signals to the two different target positions, respectively. Merely by way of example, when the output circuit includes the circuit board, one side of the circuit board may correspond to one target position, and the first sensing unit may transmit the electric signal to the one target position; another side of the circuit board may correspond to the other target position, and the second sensing unit may transmit the electric signal to the other target position. The one target position may be disposed between the first side surface 110-a and the second side surface 110-b, and the other target position may be disposed on the second side surface 110-b. More descriptions regarding the above may be found in FIG. 7 and related descriptions thereof.

Referring to FIG. 1, in some embodiments, the circuit board 151 includes a flexible region and a non-flexible region. The flexible region is disposed at the target position, and a processing circuit (e.g., the processing circuit 152 in FIG. 1) is arranged at the non-flexible region.

The flexible region refers to a region capable of elastically deforming and bending, and the non-flexible region refers to a rigid region. In some embodiments, to avoid the non-flexible region of the circuit board from hindering the deformation of the flexible substrate 110, a projection of the non-flexible region on the target object is located outside a projection of the first sensing unit 120 on the target object. For purposes of illustration, in a direction from the sensor device 100 toward the target object (i.e., the X-direction), the first sensing unit 120 has a first projection on the target object (e.g., a segment CD in FIG. 1), and the circuit board 151 has a second projection on the target object (e.g., a segment EF in FIG. 1). A region corresponding to a portion of the second projection located within the first projection (e.g., a segment ED in FIG. 1) is the flexible region of the circuit board 151, and a region corresponding to a portion of the second projection located outside the first projection (e.g., a segment DF in FIG. 1) is the non-flexible region of the circuit board 151. Merely by way of example, when the target position is located between the first side surface 110-a and the second side surface 110-b (e.g., the region A shown in FIG. 1), the flexible region is a portion of the circuit board 151 disposed on the flexible substrate 110, and the non-flexible region is a portion of the circuit board 151 disposed outside the flexible substrate 110. In some embodiments, the flexible region of the circuit board may employ an FPC. The non-flexible region may employ a rigid PCB.

The flexible region may bend and elastically deform correspondingly with the deformations of the first sensing unit 120 and the flexible substrate 110, so as to avoid hindering the deformations of the first sensing unit 120 and the flexible substrate 110 and preventing the measurement of the motion of the target object by the sensor device 100 from being inaccurate. The non-flexible region may provide a rigid support for the processing circuit 152, and provide an installation position for the processing circuit 152 while preventing the processing circuit 152 from being damaged due to bending. On the other hand, the arrangement of the flexible region and the non-flexible region may cause a stress concentration point of the circuit board 151 to be located at the non-flexible region, so that a projection of the stress concentration point of the circuit board 151 on the target object is located outside the first projection of the first sensing unit 120 on the target object. In this way, the influence of the circuit board 151 on the deformation of the first sensing unit 120 and the flexible substrate 110 can be reduced, and the operational stability and accuracy of the sensor device 100 can be improved.

In some embodiments, the non-flexible region of the circuit board 151 may further be provided with a pad 154, and the circuit board 151 may be electrically connected to the external circuit through the pad 154.

In some embodiments, the non-flexible region may further be provided with a reinforcement plate 153, and the reinforcement plate 153 may be made of a rigid material (e.g., a stainless-steel sheet or a ceramic sheet). The reinforcement plate 153 may provide a further support for the non-flexible region, and may reduce a probability that the processing circuit 152 is damaged due to bending of the non-flexible region.

By providing the processing circuit 152 on the circuit board 151, the sensor device 100 may directly process the electric signal generated by the first sensing unit 120 without additionally connecting to a processor, and the signal output by the sensor device 100 may be directly delivered to a terminal to display the motion information of the target object.

In other embodiments, the output circuit 150 may not include the processing circuit 152, and the processing circuit 152 may be connected to the output circuit 150 as the external circuit. At this time, when the output circuit 150 includes the circuit board 151, correspondingly, the circuit board 151 may not include the non-flexible region and does not need to be provided with the reinforcement plate 153. When the output circuit 150 includes the wire (e.g., the wire 655 shown in FIG. 4), the wire is directly connected to the processing circuit serving as the external circuit. In this way, the size and volume of the sensor device 100 can be effectively reduced, and the structural design of the sensor device 100 can be simplified.

In some embodiments, the sensor device 100 may further include a protective layer 130, and the protective layer 130 is disposed at an outermost layer of the sensor device 100 to provide protection for the sensor device 100 and prevent components of the sensor device 100 (e.g., the first sensing unit 120, the output circuit 150, etc.) from being contacted or corroded by an external environment.

In some embodiments, the protective layer 130 may include a first protective portion 131 and a second protective portion 132. The first protective portion 131 covers the first sensing unit 120 to protect the first sensing unit 120. The second protective portion 132 covers the output circuit 150 to protect the output circuit 150.

In some embodiments, a thickness by which the first protective portion 131 protrudes from the first side surface 110-a is the same as a thickness by which the second protective portion 132 protrudes from the first side surface 110-a. That is, a surface of the first protective portion 131 facing the target object is flush with a surface of the second protective portion 132 facing the target object, so that a side of the sensor device 100 facing the target object may remain flat, the non-uniform deformation of the first sensing unit 120 may be avoided, and the operational stability of the sensor device 100 can be improved. Meanwhile, the foreign-body sensation when the sensor device 100 is worn can be reduced, and the wearing comfort of the sensor device 100 can be improved.

In some embodiments, a thickness by which the first protective portion 131 protrudes from the second side surface 110-b may be the same as or different from a thickness by which the second protective portion 132 protrudes from the second side surface 110-b, and the present disclosure does not impose further limitations thereon.

Referring to FIG. 1, as shown in FIG. 1, the sensor device 100 only includes the first sensing unit 120 disposed on the first side surface 110-a, and the first sensing unit 120 is a capacitive structure. The target position is located between the first side surface 110-a and the second side surface 110-b, such as the region A shown in FIG. 1. The output circuit 150 includes the circuit board 151, the processing circuit 152, and the reinforcement plate 153. More descriptions regarding the output circuit 150 may be found in previous descriptions, and detailed descriptions thereof are omitted herein.

In some embodiments, in the direction from the sensor device 100 toward the target object (i.e., the X-direction), the first sensing unit 120 includes a first electrode layer 121, a first dielectric layer 122, and a second electrode layer 123 that are sequentially disposed. The first dielectric layer 122 is located between the first electrode layer 121 and the second electrode layer 123. The first electrode layer 121 is connected to the first side surface 110-a of the flexible substrate 110, and the second electrode layer 123 is connected to the first protective portion 131.

The first conductive channel 111 may include a first sub-channel 111-1 and a second sub-channel 111-2. The first electrode layer 121 is electrically connected to the first sub-channel 111-1, and the second electrode layer 123 is electrically connected to the second sub-channel 111-2, so that the first electrode layer 121 may be electrically connected to the output circuit 150 at the target position through the first sub-channel 111-1, and the second electrode layer 123 may be electrically connected to the output circuit 150 at the target position through the second sub-channel 111-2, thereby implementing a connection between the capacitive structure and the output circuit 150. It should be noted that, in some embodiments, when the first electrode layer 121 or the second electrode layer 123 of the first sensing unit 120 has a plurality of electrodes, a plurality of first sub-channels 111-1 or a plurality of second sub-channels 111-2 are provided according to the count of the corresponding electrodes. In some embodiments, when the sensor device 100 is provided with a plurality of sensing units, an electrode layer of each sensing unit may be provided with one or more electrodes, and one or more sub-channels of a corresponding conductive channel may also be provided according to the count of the one or more electrodes, so as to transmit the electric signal generated by the sensing unit to the output circuit 150.

In some embodiments, the first sub-channel 111-1 forms a first sub-port (not shown in the figure) on the first side surface 110-a. The first sub-channel 111-1 forms a second sub-port (not shown in the figure) at the target position (e.g., the region A). The first sub-channel 111-1 is electrically connected to an electrode of the first electrode layer 121 at the first sub-port. The first sub-channel 111-1 is electrically connected to the output circuit 150 at the second sub-port. The second sub-channel 111-2 forms a third sub-port (not shown in the figure) on the first side surface 110-a. The second sub-channel 111-2 forms a fourth sub-port (not shown in the figure) at the target position (e.g., the region A). The second sub-channel 111-2 is electrically connected to an electrode of the second electrode layer 123 at the third sub-port. The second sub-channel 111-2 is electrically connected to the output circuit 150 at the fourth sub-port.

As shown in FIG. 1, the thickness by which the first protective portion 131 protrudes from the second side surface 110-b may be different from the thickness by which the second protective portion 132 protrudes from the second side surface 110-b. That is, a surface of the first protective portion 131 away from the target object is not flush with a surface of the second protective portion 132 away from the target object, so that a side of the sensor device 100 away from the target object is not flat. This results in a significant a distinction between a side of the sensor device 100 provided with the first sensing unit 120 and another side not provided with a sensing unit, whereby correct wearing of the sensor device 100 can be facilitated.

In some embodiments, when the target position is disposed between the first side surface 110-a and the second side surface 110-b (e.g., the region A shown in FIG. 1), the flexible substrate 110 may have a symmetric structure centered on the circuit board 151 of the output circuit 150 disposed at the target position. At this time, the flexible substrate 110 may correspondingly include a first sub-substrate 110-1 and a second sub-substrate 110-2 located at two sides of the circuit board 151. In some embodiments, a region in which the first conductive channel 111 is located (i.e., a region in which the first sub-channel 111-1 and the second sub-channel 111-2 are located) on the first sub-substrate 110-1 is bonded to the circuit board 251 using a conductive glue, so as to ensure the electrical connection between the first conductive channel 111 and the circuit board 151. The first sub-substrate 110-1 and the second sub-substrate 110-2 are bonded using a non-conductive glue, and a region on the first sub-substrate 110-1 other than the first conductive channel 111 is bonded to the circuit board 251 using the non-conductive glue, so as to avoid an interference with the transmission of the electric signal.

In some embodiments, the first sensing unit 120 may first be prepared, and a preparation manner of the first sensing unit 120 may include, but is not limited to, spraying, sputtering, printing, etc. For example, two electrode layers may be respectively formed by printing on two films, and the capacitive structure of the first sensing unit 120 may be obtained by bonding the two films. The two electrode layers correspond to the first electrode layer 121 and the second electrode layer 123, respectively, and the two films after bonding correspond to the first dielectric layer 122. As another example, the second electrode layer 123, the first dielectric layer 122, and the first electrode layer 121 may be sequentially prepared on a surface of the first protective portion 131 of the protective layer 130 that abuts against the first side surface 110-a. Compared with the first electrode layer 121 and the first dielectric layer 122, the second electrode layer 123 is farther away from the flexible substrate 110. To avoid the first electrode layer 121 and the first dielectric layer 122 from hindering the electrical connection between the second electrode layer 123 and the second sub-channel 111-2, in some embodiments, a size of the second electrode layer 123 is greater than sizes of the first electrode layer 121 and the first dielectric layer 122, so that the second electrode layer 123 forms a protrusion toward the flexible substrate 110, and the protrusion abuts against the second sub-channel 111-2 on the flexible substrate 110 to implement the electrical connection between the second electrode layer 123 and the second sub-channel 111-2. In some embodiments, materials of the first electrode layer 121 and the second electrode layer 123 may include flexible conductive materials such as conductive ink and liquid metal.

The first sensing unit 120 is pasted onto the first side surface 110-a of the first sub-substrate 110-1, and the first hole portion is then processed on the first sub-substrate 110-1 and filled with the flexible conductive material to form the first conductive channel 111. The flexible conductive material in the first hole portion is brought into full contact with the electrode layer of the first sensing unit 120 and cured by heating and pressing, so as to implement the preparation and installation of the first sensing unit 120 and the first sub-substrate 110-1.

In some embodiments, the sensor device 100 further includes the second sensing unit 140 disposed on the second side surface 110-b. By providing the second sensing unit 140, the second sensing unit 140 may also deform with the motion of the target object and correspondingly generate an electric signal, and the electric signal generated by the second sensing unit 140 may be combined with and compared to the electric signal generated by the first sensing unit 120, so that the measurement accuracy of the sensor device 100 can be improved.

In some embodiments, the second sensing unit 140 may be a capacitive structure. In a direction from the first side surface 110-a toward the second side surface 110-b (i.e., an opposite direction of the X-direction), the second sensing unit 140 includes a third electrode layer 141, a second dielectric layer 142, and a fourth electrode layer 143 that are sequentially disposed. The second dielectric layer 142 is located between the third electrode layer 141 and the fourth electrode layer 143. The third electrode layer 141 is connected to the second side surface 110-b of the flexible substrate 110, and the fourth electrode layer 143 is connected to the first protective portion 231.

In some embodiments, the flexible substrate 110 is provided with a second conductive channel 112. The second conductive channel 112 forms a third end portion on the second side surface 110-b. The third end portion is electrically connected to the electrode of the second sensing unit 140. The second conductive channel 112 forms a fourth end portion at the target position. The fourth end portion is electrically connected to the output circuit 150 at the target position, so as to transmit the electric signal generated by the second sensing unit 140 to the output circuit 150 at the target position through the second conductive channel 112.

In some embodiments, the second conductive channel 112 includes a third sub-channel 112-1 and a fourth sub-channel 112-2. The third sub-channel 112-1 forms a fifth sub-port (not shown in the figure) on the second side surface 110-b, and the third sub-channel 112-1 forms a sixth sub-port (not shown in the figure) at the target position (e.g., the region A shown in FIG. 1). The third sub-channel 112-1 is electrically connected to an electrode of the third electrode layer 141 at the fifth sub-port, and is electrically connected to the output circuit 150 at the sixth sub-port. The fourth sub-channel 112-2 forms a seventh sub-port (not shown in the figure) on the second side surface 110-b, and the fourth sub-channel 112-2 forms an eighth sub-port (not shown in the figure) at the target position (e.g., the region A shown in FIG. 1). The fourth sub-channel 112-2 is electrically connected to an electrode of the fourth electrode layer 143 at the seventh sub-port, and the fourth sub-channel 112-2 is electrically connected to the output circuit 150 at the eighth sub-port.

In some embodiments, for the protective layer 130, the thickness by which the first protective portion 131 protrudes from the second side surface 110-b may be the same as the thickness by which the second protective portion 132 protrudes from the second side surface 110-b. That is, the surface of the first protective portion 131 away from the target object is flush with the surface of the second protective portion 132 away from the target object, so that the side of the sensor device 100 away from the target object may remain flat, the non-uniform deformation of the second sensing unit 140 may be avoided, and the operational stability of the sensor device 100 can be improved. Meanwhile, the foreign-body sensation when the sensor device 100 is worn can be reduced, and the wearing comfort of the sensor device 100 can be improved.

In some embodiments, the thickness by which the first protective portion 131 of the protective layer 130 protrudes from the second side surface 110-b may also be different from the thickness by which the second protective portion 132 protrudes from the second side surface 110-b. That is, the surface of the first protective portion 131 away from the target object is not flush with the surface of the second protective portion 132 away from the target object, this results in a significant distinction between the two sides of the sensor device 100 facing and away from the target object, whereby correct wearing of the sensor device 100 can be facilitated. Meanwhile, because the second protective portion 132 needs to cover the output circuit 150 to protect the output circuit 150, and the circuit board 151 and the processing circuit 152 of the output circuit 150 have certain thicknesses, the second protective portion 132 needs to have a certain thickness to cover the output circuit 150. The thickness of the first protective portion 131 is not particularly required. In some embodiments, compared with the first protective portion 131, the thickness by which the second protective portion 132 protrudes from the second side surface 110-b may be greater so as to cover and protect the output circuit 150.

In some embodiments, when the target position is disposed between the first side surface 110-a and the second side surface 110-b (e.g., the region A shown in FIG. 1), the flexible substrate 110 may have a symmetric structure centered on the circuit board 151 of the output circuit 150 disposed at the target position. At this time, the flexible substrate 110 may correspondingly include the first sub-substrate 110-1 and the second sub-substrate 110-2 located at the two sides of the circuit board 151. The connection between the first sub-substrate 110-1 and the first sensing unit 120 and the connection between the second sub-substrate 110-2 and the second sensing unit 140 may be made with reference to the connection between the first sub-substrate 110-1 and the first sensing unit 120, and detailed descriptions thereof are omitted herein.

In some embodiments, the preparation and installation of the first sub-substrate 110-1 with the first sensing unit 120 and the preparation and installation of the second sub-substrate 110-2 with the second sensing unit 140 may be the same as the preparation and installation of the first sub-substrate 110-1 with the first sensing unit 120, and detailed descriptions thereof are omitted herein. The circuit board 151 of the output circuit 150 is placed and bonded between the first sub-substrate 110-1 and the second sub-substrate 110-2, and finally the protective layer 130 is covered, thereby completing the preparation of the sensor device 100.

FIG. 2 is a schematic diagram illustrating another exemplary structure of a sensor device according to some embodiments of the present disclosure. As shown in FIG. 2, a sensor device 400 includes the flexible substrate 410, a first sensing unit 420, a protective layer 430, a second sensing unit 440, and an output circuit 450. A target position is located at a second side surface 410-b, e.g., the region B shown in FIG. 2. Descriptions regarding the flexible substrate 410 (e.g., a first side surface 410-a, the second side surface 410-b, etc.), the first sensing unit 420 (e.g., a first electrode layer 421, a first dielectric layer 422, a second electrode layer 423, etc.), the protective layer 430 (e.g., a first protective portion 431, a second protective portion 432, etc.), and the output circuit 450 (e.g., the circuit board 451, a processing circuit 452, a pad 454, etc.) may be the same as or similar to those of the flexible substrate 110, the first sensing unit 120, the protective layer 130, and the output circuit 150 shown in FIG. 1, and detailed descriptions thereof are omitted herein. The difference between the sensor device 400 and the sensor device 100 lies in that the target position of the sensor device 400 is located at the second side surface 410-b, and the output circuit 450 is correspondingly disposed on the second side surface 410-b, e.g., the region B shown in FIG. 2.

As shown in FIG. 2, in some embodiments, the circuit board 451 is disposed on the second side surface 410-b. The circuit board 451 has a relatively small influence on deformations of the flexible substrate 410 and the first sensing unit 420, and the circuit board 451 does not need to deform correspondingly with the deformations of the flexible substrate 410 and the first sensing unit 420. The circuit board 451 may be an FPC or a rigid PCB, and the arrangement of the circuit board 451 may be more flexible.

In some embodiments, when the circuit board 451 is a rigid PCB, the reinforcement plate may not be additionally provided.

In some embodiments, the circuit board 451 may be flush with a side of the first protective portion 431 away from the target object, and the circuit board 451 is covered by the second protective portion 432 to reduce the influence of the circuit board 451 on the first sensing unit 420 and to protect the circuit board 451 and prevent external contact and corrosion. At this time, a first sub-channel 411-1 and a second sub-channel 411-2 of the first conductive channel 411 penetrate through the flexible substrate 410 and extend to the target position. Specifically, the first sub-channel 411-1 and the second sub-channel 411-2 may correspondingly penetrate through the protective layer 430 between the circuit board 451 and the flexible substrate 410 and extend to the circuit board 451 at the target position, so as to transmit the electric signal generated by the first sensing unit 420 to the output circuit 450 at the target position.

In some embodiments, a third electrode layer 441 and a fourth electrode layer 443 of the second sensing unit 440 may be directly and electrically connected to the output circuit 450 (e.g., the circuit board 451) disposed on the second side surface 410-b, without additionally providing a conductive channel, so that the structure of the sensor device 400 can be simplified.

In some embodiments, the preparation and installation of the flexible substrate 410 with the first sensing unit 420 may be the same as the preparation and installation of the first sub-substrate 110-1 with the first sensing unit 120, and detailed descriptions thereof are omitted herein. As shown in FIG. 2, because the third electrode layer 441 and the fourth electrode layer 443 of the second sensing unit 440 are connected to the circuit board 451 of the output circuit 450 after penetrating through the first protective portion 431 of the protective layer 430, a process for directly preparing the second sensing unit 440 on a surface of the first protective portion 431 of the protective layer 430 that contacts the second side surface 410-b is relatively difficult and involves complicated steps. In some embodiments, the second sensing unit 440 may be prepared by sequentially printing the third electrode layer 441, the second dielectric layer 442, and the fourth electrode layer 443 on the second side surface 410-b of the flexible substrate 410 through a screen-printing manner. Compared with the fourth electrode layer 443 and the second dielectric layer 442, the third electrode layer 441 is farther away from the output circuit 450 (e.g., the circuit board 451). To avoid the fourth electrode layer 443 and the second dielectric layer 442 from hindering the electrical connection between the third electrode layer 441 and the output circuit 450, in some embodiments, a size of the third electrode layer 441 is greater than sizes of the fourth electrode layer 443 and the second dielectric layer 442, so that the third electrode layer 441 forms a protrusion toward the output circuit 450 (e.g., the circuit board 451), and the protrusion abuts against the output circuit 450 (e.g., the circuit board 451) to implement the electrical connection between the second electrode layer 123 and the second sub-channel 111-2.

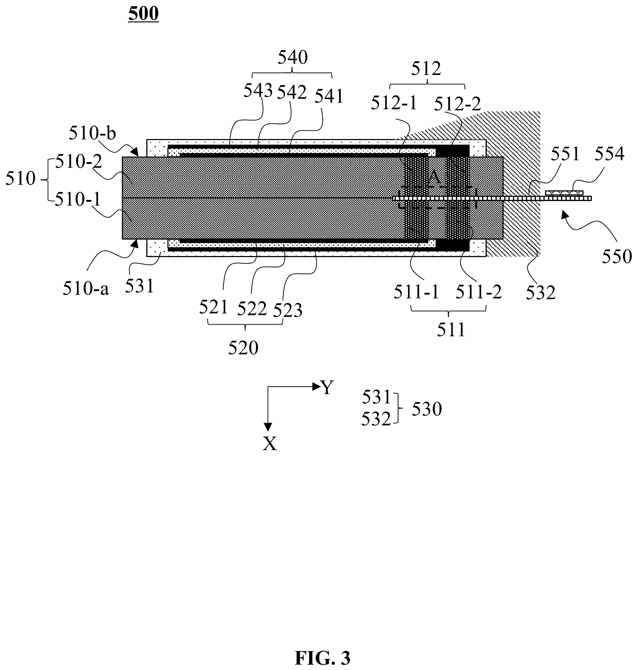

FIG. 3 is a schematic diagram illustrating another exemplary structure of a sensor device according to some embodiments of the present disclosure. As shown in FIG. 3, a sensor device 500 includes a flexible substrate 510, a first sensing unit 520, a protective layer 530, a second sensing unit 540, and an output circuit 550. A target position is located between a first side surface 510-a and a second side surface 510-b, e.g., the region A shown in FIG. 3. Descriptions regarding the flexible substrate 510 (e.g., a first sub-substrate 510-1, a second sub-substrate 510-2, a first sub-channel 511-1 and a second sub-channel 511-2 of a first conductive channel 511, a third sub-channel 512-1 and a fourth sub-channel 512-2 of a second conductive channel 512, the first side surface 510-a, the second side surface 510-b, etc.), the first sensing unit 520 (e.g., a first electrode layer 521, a first dielectric layer 522, a second electrode layer 523, etc.), the protective layer 530 (e.g., a first protective portion 531, a second protective portion 532, etc.), and the second sensing unit 540 (e.g., a third electrode layer 541, a second dielectric layer 542, a fourth electrode layer 543, etc.) may be the same as or similar to those of the flexible substrate 110, the first sensing unit 120, the protective layer 130, and the second sensing unit 140 shown in FIG. 1, and detailed descriptions thereof are omitted herein. The difference between the sensor device 500 and the sensor device 100 lies in that the output circuit 550 of the sensor device 500 is not provided with the processing circuit or the reinforcement plate. That is, the output circuit 550 does not process the electric signal generated by the first sensing unit 520 or the electric signal generated by the second sensing unit 540, but directly transmits the electric signals to an external circuit connected to a pad 554 through a circuit board 551, and the external circuit performs processing and analysis on the electric signals.

In some embodiments, a thickness by which the first protective portion 531 of the protective layer 530 of the sensor device 500 protrudes from the second side surface 510-b may be different from a thickness by which the second protective portion 532 protrudes from the second side surface 510-b. That is, a surface of the first protective portion 531 away from the target object is not flush with a surface of the second protective portion 532 away from the target object, this results in a significant distinction between two sides of the sensor device 500 facing and away from the target object, whereby correct wearing of the sensor device 500 can be facilitated.

FIG. 4 is a schematic diagram illustrating another exemplary structure of a sensor device according to some embodiments of the present disclosure. As shown in FIG. 4, a sensor device 600 includes a flexible substrate 610, a first sensing unit 620, a protective layer 630, a second sensing unit 640, and an output circuit 650. A target position is located at a second side surface 610-b, e.g., the region B shown in FIG. 4. Descriptions regarding the flexible substrate 610 (e.g., a first side surface 610-a, a second side surface 610-b, a first sub-channel 611-1 and a second sub-channel 611-2 of a first conductive channel 611, etc.), the first sensing unit 620 (e.g., a first electrode layer 621, a first dielectric layer 622, a second electrode layer 623, etc.), the protective layer 630 (e.g., a first protective portion 631, a second protective portion 632, etc.), and the second sensing unit 640 (e.g., a third electrode layer 641, a second dielectric layer 642, a fourth electrode layer 643, etc.) may be the same as or similar to those of the flexible substrate 410, the first sensing unit 420, the protective layer 430, and the second sensing unit 440 shown in FIG. 2, and detailed descriptions thereof are omitted herein. The difference between the sensor device 600 and the sensor device 400 lies in that the output circuit 650 of the sensor device 600 is the wire 655. That is, the output circuit 650 does not process the electric signal generated by the first sensing unit 620 or the electric signal generated by the second sensing unit 640, but directly transmits the electric signals to an external circuit connected to the wire 655 through the wire 655, and the external circuit performs processing and analysis on the electric signals.

In some embodiments, the first electrode layer 621 of the first sensing unit 620 may be electrically connected to a first wire through the first sub-channel 611-1, the second electrode layer 623 of the first sensing unit 620 may be electrically connected to a second wire through the second sub-channel 611-2, the third electrode layer 641 of the second sensing unit 640 may be electrically connected to a third wire, and the fourth electrode layer 643 of the second sensing unit 640 may be electrically connected to a fourth wire. The four wires are electrically connected to the external circuit together.

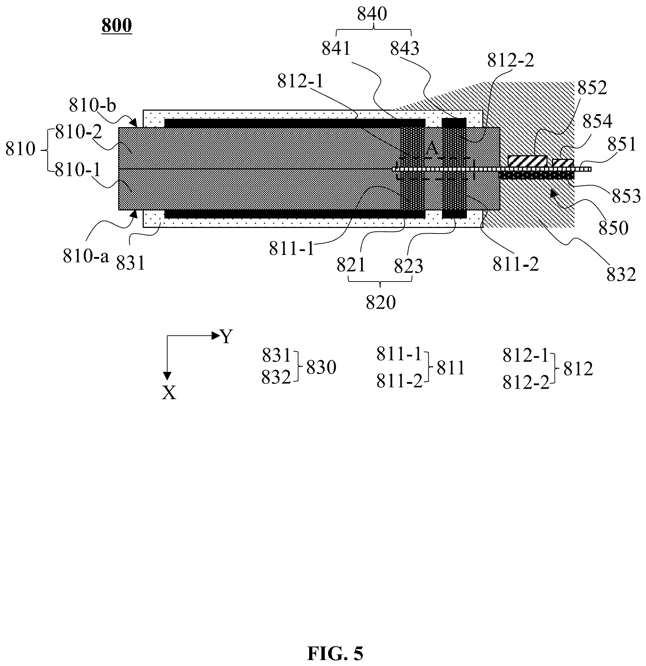

FIG. 5 is a schematic diagram illustrating another exemplary structure of a sensor device according to some embodiments of the present disclosure. As shown in FIG. 5, a sensor device 800 includes a flexible substrate 810, a first sensing unit 820, a protective layer 830, a second sensing unit 840, and an output circuit 850. A target position is located between a first side surface 810-a and a second side surface 810-b, e.g., the region A shown in FIG. 5. Descriptions regarding the flexible substrate 810 (e.g., a first sub-substrate 810-1, a second sub-substrate 810-2, a first sub-channel 811-1 and a second sub-channel 811-2 of a first conductive channel 811, a third sub-channel 812-1 and a fourth sub-channel 812-2 of a second conductive channel 812, the first side surface 810-a, the second side surface 810-b, etc.), the protective layer 830 (e.g., a first protective portion 831, a second protective portion 832, etc.), and the output circuit 850 (e.g., a circuit board 851, a processing circuit 852, a reinforcement plate 853, a pad 854, etc.) may be the same as or similar to those of the flexible substrate 110, the protective layer 130, and the output circuit 150, and detailed descriptions thereof are omitted herein. The difference between the sensor device 800 and the sensor device 100 lies in that the first sensing unit 820 and the second sensing unit 840 may be resistive structures or inductive structures.

In some embodiments, the first sensing unit 820 may include a first electrode 821 and a second electrode 823. The first sub-channel 811-1 is electrically connected to the first electrode 821 at a first sub-port on the first side surface 810-a, and the first sub-channel 811-1 is electrically connected to the output circuit 850 at a second sub-port at the target position (e.g., the region A shown in FIG. 5). The second sub-channel 811-2 is electrically connected to the second electrode 823 at a third sub-port on the first side surface 810-a, and the second sub-channel 811-2 is electrically connected to the output circuit 850 at a fourth sub-port at the target position (e.g., the region A shown in FIG. 5). The second sensing unit 840 may include a third electrode 841 and a fourth electrode 843. The flexible substrate 810 may be provided with the second conductive channel 812, and the third electrode 841 and the fourth electrode 843 of the second sensing unit 840 are respectively electrically connected to the output circuit 850 through the second conductive channel 812. For example, the second conductive channel 812 may include the third sub-channel 812-1 and the fourth sub-channel 812-2. The third sub-channel 812-1 forms a fifth sub-port on the second side surface 810-b, and the third sub-channel 812-1 forms a sixth sub-port at the target position (e.g., the region A shown in FIG. 5). The third sub-channel 812-1 is electrically connected to the third electrode 841 at the fifth sub-port, and the third sub-channel 812-1 is electrically connected to the output circuit 850 at the sixth sub-port. The fourth sub-channel 812-2 forms a seventh sub-port on the second side surface 810-b, and the fourth sub-channel 812-2 forms an eighth sub-port at the target position (e.g., the region A shown in FIG. 5). The fourth sub-channel 812-2 is electrically connected to the fourth electrode 843 at the seventh sub-port, and the fourth sub-channel 812-2 is electrically connected to the output circuit 850 at the eighth sub-port.

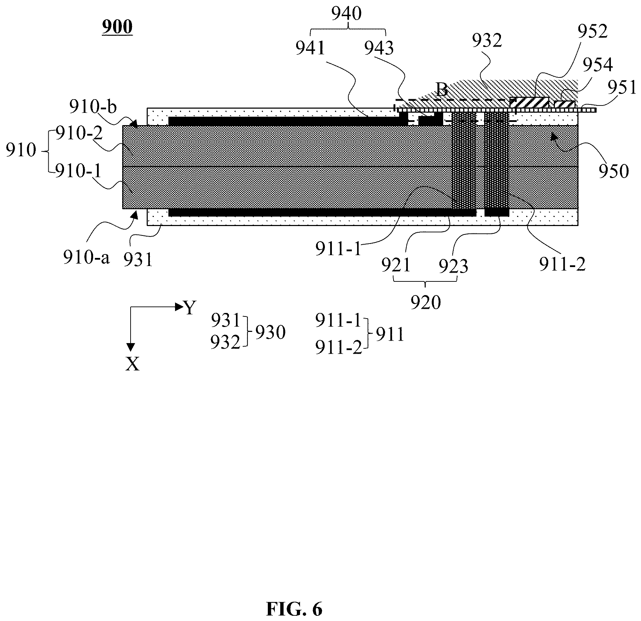

FIG. 6 is a schematic diagram illustrating another exemplary structure of a sensor device according to some embodiments of the present disclosure. As shown in FIG. 6, a sensor device 900 includes a flexible substrate 910, a first sensing unit 920, a protective layer 930, a second sensing unit 940, and an output circuit 950. Descriptions regarding the flexible substrate 910 (e.g., a first sub-substrate 810-1, a second sub-substrate 810-2, a first side surface 810-a, a second side surface 810-b, etc.), the first sensing unit 920, the protective layer 930 (e.g., a first protective portion 931, a second protective portion 932, etc.), the second sensing unit 940, and the output circuit 950 (e.g., a circuit board 951, a processing circuit 952, a pad 954, etc.) may be the same as or similar to those of the flexible substrate 810, the first sensing unit 820, the protective layer 830, the second sensing unit 840, and the output circuit 850, and detailed descriptions thereof are omitted herein. The difference between the sensor device 900 and the sensor device 800 lies in that a target position of the sensor device 900 is disposed on the second side surface 910-b, e.g., the region B shown in FIG. 6, and the output circuit 950 is correspondingly disposed on the second side surface 910-b.

In some embodiments, when the circuit board 951 of the output circuit 950 is disposed on the second side surface 910-b, the circuit board 951 has a relatively small influence on deformations of the flexible substrate 910, the first sensing unit 920, and the second sensing unit 940, and the circuit board 951 does not need to deform correspondingly with deformations of the flexible substrate 910, the first sensing unit 920, and the second sensing unit 940. The circuit board 951 may be an FPC or a rigid PCB, and the arrangement of the circuit board 951 may be more flexible. In some embodiments, when the circuit board 951 is a rigid PCB, a reinforcement plate may not be additionally provided.

In some embodiments, a third electrode 941 and a fourth electrode 943 of the second sensing unit 940 may be directly and electrically connected to the output circuit 950 (e.g., the circuit board 951) disposed on the second side surface 910-b, without additionally providing a conductive channel, so that the structure of the sensor device 900 can be simplified.

FIG. 7 is a schematic diagram illustrating another exemplary structure of a sensor device according to some embodiments of the present disclosure. As shown in FIG. 7, a sensor device 1000 includes a flexible substrate 1010, a first sensing unit 1020, a protective layer 1030, a second sensing unit 1040, and an output circuit 1050. Descriptions regarding the first sensing unit 1020 (e.g., a first electrode layer 1021, a first dielectric layer 1022, a second electrode layer 1023, etc.), the protective layer 1030 (e.g., a first protective portion 1031, a second protective portion 1032, etc.), and the second sensing unit 1040 (e.g., a third electrode layer 1041, a second dielectric layer 1042, a fourth electrode layer 1043, etc.) may be the same as or similar to those of the first sensing unit 120, the protective layer 130, and the second sensing unit 140 of the sensor device 100 shown in FIG. 1, or the first sensing unit 420, the protective layer 430, and the second sensing unit 440 of the sensor device 400 shown in FIG. 2, and detailed descriptions thereof are omitted herein. The difference between the sensor device 1000 and the sensor device 100 as well as the sensor device 400 lies in that a target position of the first sensing unit 1020 of the sensor device 1000 is located between a first side surface 1010-a and a second side surface 1010-b, e.g., the region A shown in FIG. 7, and a target position of the second sensing unit 1040 is located at the second side surface 1010-b, e.g., the region B shown in FIG. 7. The output circuit 1050 may be electrically connected to the corresponding sensing units at the two target positions, respectively.

In some embodiments, with a plane passing through the region A and parallel to the first side surface 1010-a or the second side surface 1010-b as a boundary, the flexible substrate 1010 may be divided into a first sub-substrate 1010-1 close to the target object and a second sub-substrate 1010-2 away from the target object. A side of the first sub-substrate 1010-1 close to the target object is the first side surface 1010-a, and a side of the second sub-substrate 1010-2 away from the target object is the second side surface 1010-b. Along a direction from the sensor device 1000 toward the target object (i.e., the X-direction), a thickness of the first sub-substrate 1010-1 and a thickness of the second sub-substrate 1010-2 may be the same or different. Along an extending direction of the flexible substrate 1010 (i.e., the Y-direction), a length of the first sub-substrate 1010-1 may be smaller than a length of the second sub-substrate 1010-2. In some embodiments, the first sub-substrate 1010-1 and the second sub-substrate 1010-2 may be integrally formed or may be separately prepared and then bonded.

In some embodiments, a circuit board 1051 of the output circuit 1050 may be arranged side by side with the second sub-substrate 1010-2 in the Y-direction on a side of the first sub-substrate 1010-1 opposite to the first side surface 1010-a, so that one side of the output circuit 1050 is electrically connected to the first sensing unit 1020 at the region A through a first conductive channel 1011, and the other side of the output circuit 1050 is directly electrically connected to the second sensing unit 1040 at the region B. A processing circuit 1052 and a pad 1054 are both disposed on the other side of the circuit board 1051.

In some embodiments, a thickness of the circuit board 1051 of the output circuit 1050 may be the same as or approximately the same as a thickness of the second sub-substrate 1010-2, i.e., a side of the second sub-substrate 1010-2 away from the target object and a side of the circuit board 1051 away from the target object are flush or approximately flush with each other to jointly form the second side surface 1010-b. Such an arrangement can increase the thickness of the output circuit 1050 in the X-direction, thereby improving a supporting strength of the output circuit 1050 for the processing circuit 1052, and enhancing the operational stability of the sensor device 1000.

Merely by way of example, when the first sensing unit 1020 and the second sensing unit 1040 are the capacitive structures, the first conductive channel 1011 is provided on the first sub-substrate 1010-1. The first conductive channel 1011 includes a first sub-channel 1011-1 and a second sub-channel 1011-2. The first electrode layer 1021 of the first sensing unit 1020 is electrically connected to the first sub-channel 1011-1 at a first sub-port on the first side surface 1010-a, and the first sub-channel 1011-1 is electrically connected to one side of the circuit board 1051 at a second sub-port at the region A. The second electrode layer 1023 of the first sensing unit 1020 is electrically connected to the second sub-channel 1011-2 at a third sub-port on the first side surface 1010-a, and the second sub-channel 1011-2 is electrically connected to the one side of the circuit board 1051 at a fourth sub-port at the region A. The third electrode layer 1041 and the fourth electrode layer 1043 of the second sensing unit 1040 are directly and electrically connected to the other side of the circuit board 1051.

The sensor device 1000 shown in FIG. 7 can simplify installation procedures, and the preparation process of the first sensing unit 1020 and the second sensing unit 1040 may be the same as the preparation process of the first sensing unit 120 and the second sensing unit 140 shown in FIG. 1, so that a yield of the first sensing unit 1020 and the second sensing unit 1040 may be relatively high. The sensor device 1000 is applicable not only to application scenarios such as laboratories but also to application scenarios of industrial mass production.

In some embodiments, when the first sensing unit 1020 and the second sensing unit 1040 are resistive structures or inductive structures, the connection manner of the first sensing unit 1020 and the second sensing unit 1040 with the circuit board 1051 may refer to the above connection manner of the capacitive structure with the circuit board 1051, and detailed descriptions thereof are omitted herein.

In some embodiments, when the output circuit 1050 includes a plurality of wires, two of the plurality of wires may be electrically connected to the first sensing unit 1020 at the region A through the first sub-channel 1011-1 and the second sub-channel 1011-2, respectively, and another two of the plurality of wires may be directly and electrically connected to the second sensing unit 1040 at the region B, respectively.

Some embodiments of the present disclosure further provide a wearable device for acquiring a motion signal of a target object. The wearable device includes the sensor device described above (e.g., the sensor device 100, the sensor device 400, the sensor device 500, the sensor device 1000, etc.), so that the wearable device may have relatively high operational stability and wearing comfort.

The basic concepts have been described above. Obviously, to those skilled in the art, the above detailed disclosure is merely an example and does not constitute a limitation to the present disclosure. Although not explicitly stated herein, those skilled in the art may make various modifications, improvements, and amendments to the present disclosure. Such modifications, improvements, and amendments are suggested in the present disclosure. Therefore, such modifications, improvements, and amendments still fall within the spirit and scope of the exemplary embodiments of the present disclosure.

Meanwhile, the present disclosure uses specific words to describe the embodiments of the present disclosure. For example, "one embodiment," "an embodiment," and/or "some embodiments" mean a certain feature, structure, or characteristic related to at least one embodiment of the present disclosure. Therefore, it should be emphasized and noted that "an embodiment" or "one embodiment" or "an alternative embodiment" mentioned two or more times in different places in the present disclosure does not necessarily refer to the same embodiment. In addition, certain features, structures, or characteristics in one or more embodiments of the present disclosure may be appropriately combined.

Similarly, it should be noted that in order to simplify the expressions disclosed in the present disclosure and thereby help understand one or more inventive embodiments, sometimes multiple features are incorporated into one embodiment, drawing, or description thereof in the foregoing description of the embodiments of the present disclosure. However, this disclosure method does not mean that the object of the present disclosure requires more features than those mentioned in the claims. Rather, claimed subject matter may lie in less than all features of a single foregoing disclosed embodiment.

Finally, it should be understood that the embodiments described in the present disclosure are only used to illustrate the principles of the embodiments of the present disclosure. Other variations may also fall within the scope of the present disclosure. Therefore, by way of example and not limitation, alternative configurations of the embodiments of the present disclosure may be considered consistent with the teachings of the present disclosure. Accordingly, the embodiments of the present disclosure are not limited to the embodiments explicitly introduced and described in the present disclosure.

## Claims

1. A sensor device for acquiring a motion signal of a target object, comprising:
a flexible substrate including a first side surface facing the target object; and
a first sensing unit, configured to detect the target object and generate an electric signal, the first sensing unit being disposed on the first side surface of the flexible substrate;
wherein the flexible substrate is provided with a first conductive channel, the first conductive channel is configured to transmit the electric signal generated by the first sensing unit to a target position, and the target position is farther away from the target object than the first side surface.

2. The sensor device of claim 1, wherein the flexible substrate is provided with a first hole portion, and the first hole portion is filled with a flexible conductive material to form the first conductive channel.

3. The sensor device of claim 1 or 2, further comprising an output circuit disposed at the target position, wherein:
the first conductive channel forms a first end portion on the first side surface, and the first end portion is electrically connected to an electrode of the first sensing unit; the first conductive channel forms a second end portion at the target position, and the second end portion is electrically connected to the output circuit.

4. The sensor device of claim 3, wherein the flexible substrate includes a second side surface opposite to the first side surface; a second sensing unit is disposed on the second side surface, and an electric signal generated by the second sensing unit is transmitted to the output circuit.

5. The sensor device of claim 4, wherein the target position is located between the first side surface and the second side surface; the flexible substrate is provided with a second conductive channel; the second conductive channel forms a third end portion on the second side surface, and the third end portion is electrically connected to an electrode of the second sensing unit; the second conductive channel forms a fourth end portion at the target position, and the fourth end portion is electrically connected to the output circuit.

6. The sensor device of claim 4, wherein the target position is located on the second side surface, and the first conductive channel penetrates through the flexible substrate and extends to the target position.

7. The sensor device of any one of claims 3 to 6, wherein the output circuit includes a circuit board or a wire.

8. The sensor device of claim 7, wherein the circuit board includes a flexible region and a non-flexible region; the flexible region is disposed at the target position, and a processing circuit is arranged on the non-flexible region.

9. The sensor device of claim 8, wherein the non-flexible region is provided with a reinforcement plate, and the reinforcement plate is attached to the non-flexible region.

10. The sensor device of claim 8, wherein a projection of the non-flexible region on the target object is located outside a projection of the first sensing unit on the target object.

11. The sensor device of any one of claims 7 to 10, wherein the flexible substrate includes a first sub-substrate and a second sub-substrate; the first sub-substrate is close to the target object, and the second sub-substrate is away from the target object; a side of the first sub-substrate close to the target object is the first side surface, and a side of the second sub-substrate away from the target object is the second side surface; and the first conductive channel is provided on the first sub-substrate; and
when the output circuit includes the circuit board, the circuit board is arranged side by side with the second sub-substrate on a side of the first sub-substrate opposite to the first side surface.

12. The sensor device of claim 1, wherein the first sensing unit is a capacitive structure; the first sensing unit includes a first electrode layer and a second electrode layer, and a dielectric layer is disposed between the first electrode layer and the second electrode layer; the first conductive channel includes a first sub-channel and a second sub-channel; the first sub-channel is electrically connected to the first electrode layer, and the second sub-channel is electrically connected to the second electrode layer.

13. The sensor device of claim 12, further comprising an output circuit disposed at the target position, wherein the first sub-channel forms a first sub-port on the first side surface, and the first sub-channel forms a second sub-port at the target position; the first sub-channel is electrically connected to an electrode of the first electrode layer at the first sub-port, and the first sub-channel is electrically connected to the output circuit at the second sub-port; and
the second sub-channel forms a third sub-port on the first side surface, and the second sub-channel forms a fourth sub-port at the target position; the second sub-channel is electrically connected to an electrode of the second electrode layer at the third sub-port, and the second sub-channel is electrically connected to the output circuit at the fourth sub-port.

14. The sensor device of claim 12, further comprising an output circuit disposed at the target position, wherein the flexible substrate includes a second side surface opposite to the first side surface; a second sensing unit is disposed on the second side surface; the second sensing unit is another capacitive structure; the second sensing unit includes a third electrode layer and a fourth electrode layer, and another dielectric layer is disposed between the third electrode layer and the fourth electrode layer; the target position is located between the first side surface and the second side surface; the flexible substrate is provided with a second conductive channel; and the second conductive channel includes a third sub-channel and a fourth sub-channel;
the third sub-channel forms a fifth sub-port on the second side surface, and the third sub-channel forms a sixth sub-port at the target position; the third sub-channel is electrically connected to an electrode of the third electrode layer at the fifth sub-port, and the third sub-channel is electrically connected to the output circuit at the sixth sub-port; and
the fourth sub-channel forms a seventh sub-port on the second side surface, and the fourth sub-channel forms an eighth sub-port at the target position; the fourth sub-channel is electrically connected to an electrode of the fourth electrode layer at the seventh sub-port, and the fourth sub-channel is electrically connected to the output circuit at the eighth sub-port.

15. The sensor device of claim 12, further comprising an output circuit disposed at the target position, wherein the flexible substrate includes a second side surface opposite to the first side surface; a second sensing unit is disposed on the second side surface; the second sensing unit is a capacitive structure; the second sensing unit includes a third electrode layer and a fourth electrode layer, and another dielectric layer is disposed between the third electrode layer and the fourth electrode layer; the target position is located on the second side surface; and the third electrode layer and the fourth electrode layer are directly and electrically connected to the output circuit, respectively.

16. The sensor device of claim 1, wherein the first sensing unit is a resistive structure or an inductive structure; the resistive structure or the inductive structure includes a first electrode and a second electrode; the first conductive channel includes a first sub-channel and a second sub-channel; the first sub-channel is electrically connected to the first electrode, and the second sub-channel is electrically connected to the second electrode.

17. The sensor device of claim 16, further comprising an output circuit disposed at the target position, wherein the first sub-channel forms a first sub-port on the first side surface, and the first sub-channel forms a second sub-port at the target position; the first sub-channel is connected to the first electrode at the first sub-port, and the first sub-channel is electrically connected to the output circuit at the second sub-port; and
the second sub-channel forms a third sub-port on the first side surface, and the second sub-channel forms a fourth sub-port at the target position; the second sub-channel is electrically connected to the second electrode at the third sub-port, and the second sub-channel is electrically connected to the output circuit at the fourth sub-port.

18. The sensor device of claim 16, further comprising an output circuit disposed at the target position, wherein the flexible substrate includes a second side surface opposite to the first side surface; a second sensing unit is disposed on the second side surface; the second sensing unit is another resistive structure or another inductive structure; the second sensing unit includes a third electrode and a fourth electrode; the target position is located between the first side surface and the second side surface; the flexible substrate is provided with a second conductive channel; the second conductive channel includes a third sub-channel and a fourth sub-channel;
the third sub-channel forms a fifth sub-port on the second side surface, and the third sub-channel forms a sixth sub-port at the target position; the third sub-channel is electrically connected to the third electrode at the fifth sub-port, and the third sub-channel is electrically connected to the output circuit at the sixth sub-port; and
the fourth sub-channel forms a seventh sub-port on the second side surface, and the fourth sub-channel forms an eighth sub-port at the target position; the fourth sub-channel is electrically connected to the fourth electrode at the seventh sub-port, and the fourth sub-channel is electrically connected to the output circuit at the eighth sub-port.

19. The sensor device of claim 16, further comprising an output circuit disposed at the target position, wherein the flexible substrate includes a second side surface opposite to the first side surface; a second sensing unit is disposed on the second side surface; the second sensing unit is another resistive structure or another inductive structure; the second sensing unit includes a third electrode and a fourth electrode; the target position is located on the second side surface; and the third electrode and the fourth electrode are directly and electrically connected to the output circuit, respectively.

20. The sensor device of claim 1, further comprising a protective layer, wherein the protective layer includes a first protective portion and a second protective portion; the first protective portion covers the first sensing unit; the sensor device further comprises an output circuit disposed at the target position; the second protective portion covers the output circuit; and a thickness by which the first protective portion protrudes from the first side surface is the same as a thickness by which the second protective portion protrudes from the first side surface.

21. A wearable device for acquiring a motion signal of a target object, comprising the sensor device of any one of claims 1 to 20.
